# EUROPEAN PATENT APPLICATION

(11) **EP 4 631 431 A2**
(43) Date of publication of application: **15.10.2025**
(21) Application number: 25198564.4
(22) Date of filing: 17.05.2021
(51) Int. Cl.: A61B 5/363

(54) **PHYSIOLOGICAL INFORMATION ACQUISITION DEVICE, PROCESSING DEVICE, AND NON-TRANSITORY COMPUTER-READABLE MEDIUM**

(30) Priority: 25.05.2020 JP 2020090725; 14.05.2021 JP 2021082437
(62) Divisional of application: 21728660.8
(71) Applicant: Nihon Kohden Corporation, Tokyo 161-8560 (JP)
(72) Inventor: KAWASHIMA, Takuya, Tokorozawa-shi, Saitama, 359-0037 (JP); SANO, Hiroto, Tokorozawa-shi, Saitama, 359-0037 (JP); MATUZAWA, Wataru, Tokorozawa-shi, Saitama, 359-0037 (JP)
(74) Representative: Grünecker Patent- und Rechtsanwälte PartG mbB

(57) **Abstract**

A physiological information acquisition device comprises a reception interface configured to receive waveform data corresponding to a measured waveform of a physiological parameter of a subject from a sensor; a notifier configured to output an alarm indicating an abnormality of at least one of a value and the measured waveform of the physiological parameter; a processor configured to cause the notifier to output the alarm based on the waveform data; and a predictor configured to predict, based on the waveform data, a probability that the physiological parameter is erroneously calculated. The processor is configured to control an output of the alarm in a case where the probability exceeds a threshold value.

## Description

### [Technical Field]

The presently disclosed subject matter relates to a device configured to acquire physiological information of a subject through a sensor. The presently disclosed subject matter also relates to a processing device configured to process the physiological information, and a non-transitory computer-readable medium having stored a computer program adapted to be executed by a processor of the processing device.

### [Background]

The physiological information acquisition device disclosed in Japanese Patent Publication No. 2018-102671A receives waveform data corresponding to a measured waveform of a physiological parameter of a subject. The physiological information acquisition device outputs an alarm indicating abnormality of at least one of the value and the measured waveform of the physiological parameter based on the waveform data. The physiological information acquisition device also outputs an alarm indicating an abnormality of the information acquisition state based on the waveform data.

### [Summary]

### [Technical Problem]

It is demanded to suppress the occurrence of a situation where a user is forced to take an unnecessary action by an alarm.

### [Solution to Problem]

In order to meet the above demand, a first illustrative aspect of the presently disclosed subject matter provides a physiological information acquisition device, comprising:
a reception interface configured to receive waveform data corresponding to a measured waveform of a physiological parameter of a subject from a sensor;
a notifier configured to output an alarm indicating that the physiological parameter is not normally acquired;
a processor configured to cause the notifier to output the alarm based on the waveform data; and
a predictor configured to predict, based on the waveform data, a probability that the physiological parameter is erroneously calculated,
wherein the processor is configured to cause the notifier to perform a notification of at least one of a quality of the waveform data, a state of the sensor, and an action shall be taken by a user, based on the probability.

In order to meet the above demand, a second illustrative aspect of the presently disclosed subject matter provides a processing device configured to process physiological information of a subject, comprising:
a reception interface configured to receive waveform data corresponding to a measured waveform of a physiological parameter of the subject from a sensor;
a processor configured to cause, based on the waveform data, an output device to output an alarm indicating that the physiological parameter is not normally acquired; and
a predictor configured to predict, based on the waveform data, a probability that the physiological parameter is erroneously calculated,
wherein the processor is configured to cause the output device to perform a notification of at least one of a quality of the waveform data, a state of the sensor, and an action shall be taken by a user, based on the probability.

In order to meet the above demand, a third illustrative aspect of the presently disclosed subject matter provides a non-transitory computer-readable medium having stored a computer program adapted to be executed by a processor of a processing device configured to process physiological information of a subject, the computer program being configured to, when executed, the processing device to:
receive waveform data corresponding to a measured waveform of a physiological parameter of the subject from a sensor;
cause, based on the waveform data, an output device to output an alarm indicating that the physiological parameter is not normally acquired;
predict, based on the waveform data, a probability that the physiological parameter is erroneously calculated; and
cause the output device to perform a notification of at least one of a quality of the waveform data, a state of the sensor, and an action shall be taken by a user, based on the probability.

The inventors of the present application have found that, in a state where an alarm indicating that the physiological parameter is not normally acquired is outputted, there is a case where the probability that the value of the physiological parameter is erroneously calculated based on the measured waveform of the physiological parameter changes with higher values.

According to the configuration of each of the first to third illustrative aspects, by referring to the probability that is predicted by the predictor, it is possible to predict a sign reaching a state where alarms are frequently outputted, thereby prompting the user to take any suitable actions. Accordingly, it is possible to suppress the occurrence of a situation where the user is forced to take an unnecessary action by an alarm.

In order to meet the above demand, a fourth illustrative aspect of the presently disclosed subject matter provides a physiological information acquisition device, comprising:
a reception interface configured to receive waveform data corresponding to a measured waveform of a physiological parameter of a subject from a sensor;
a notifier configured to output an alarm indicating an abnormality of at least one of a value and the measured waveform of the physiological parameter;
a processor configured to cause the notifier to output the alarm based on the waveform data; and
a predictor configured to predict, based on the waveform data, a probability that the physiological parameter is erroneously calculated,
wherein the processor is configured to control an output of the alarm in a case where the probability exceeds a threshold value.

In order to meet the above demand, a fifth illustrative aspect of the presently disclosed subject matter provides a processing device configured to process physiological information of a subject, comprising:
a reception interface configured to receive waveform data corresponding to a measured waveform of a physiological parameter of a subject from a sensor;
a processor configured to cause, based on the waveform data, an output device to output an alarm indicating an abnormality of at least one of a value and the measured waveform of the physiological parameter; and
a predictor configured to predict, based on the waveform data, a probability that the physiological parameter is erroneously calculated,
wherein the processor is configured to control an output of the alarm in a case where the probability exceeds a threshold value.

In order to meet the above demand, a sixth illustrative aspect of the presently disclosed subject matter provides a non-transitory computer-readable medium having stored a computer program adapted to be executed by a processor of a processing device configured to process physiological information of a subject, the computer program being configured to, when executed, the processing device to:
receive waveform data corresponding to a measured waveform of a physiological parameter of a subject from a sensor;
cause, based on the waveform data, an output device to output an alarm indicating an abnormality of at least one of a value and the measured waveform of the physiological parameter;
predict, based on the waveform data, a probability that the physiological parameter is erroneously calculated; and
control an output of the alarm in a case where the probability exceeds a threshold value.

Even when any abnormality is observed in the waveform data that is received by the reception interface, in a case where the probability that the physiological parameter that is erroneously calculated that is predicted by the predictor based on the same waveform data is high, it is highly probable that probability that the abnormality is erroneously detected. According to the configuration of each of the fourth to sixth illustrative aspects that controls the alarm to be outputted by the notifier in such a case, it is possible to cause a user to recognize that there is a possibility that an abnormality is erroneously detected. Accordingly, it is possible to suppress the occurrence of a situation where the user is forced to take an unnecessary action by an alarm.

### [Brief Description of Drawings]

[Fig. 1]

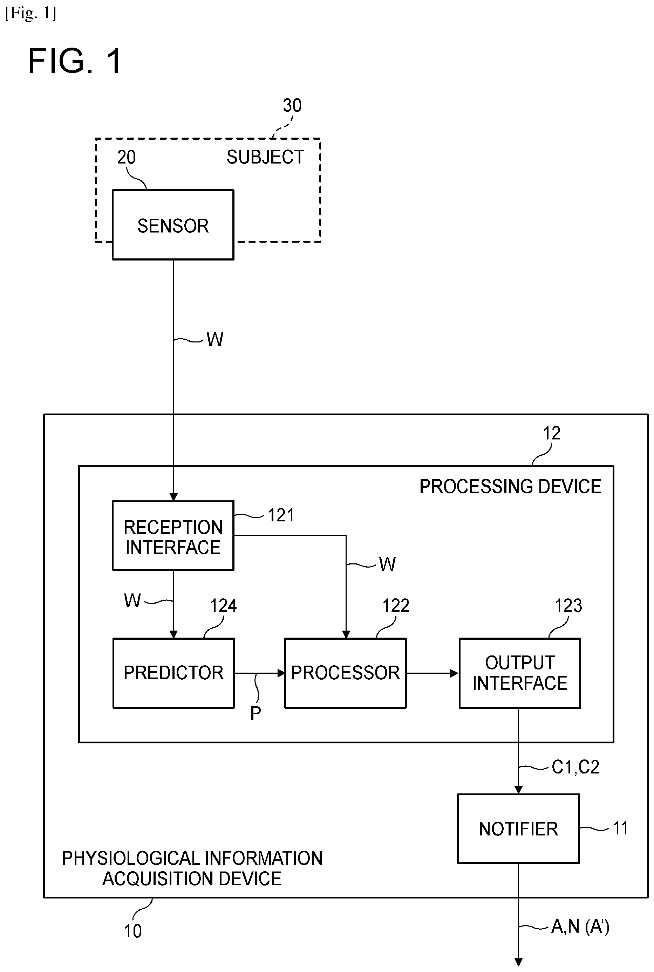

   FIG. 1 illustrates a functional configuration of a physiological information acquisition device according to one embodiment.
[Fig. 2]

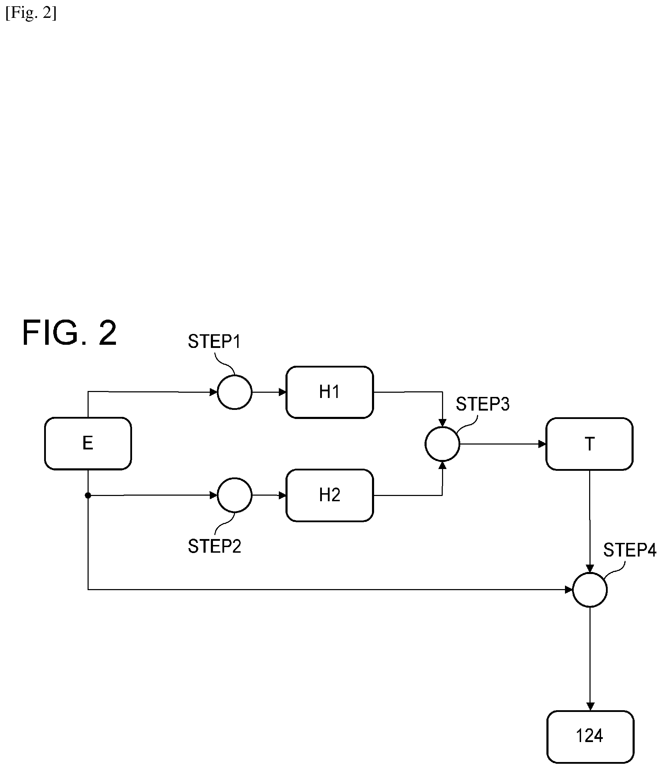

   FIG. 2 illustrates a method of creating the predictor of FIG. 1.
[Fig. 3]

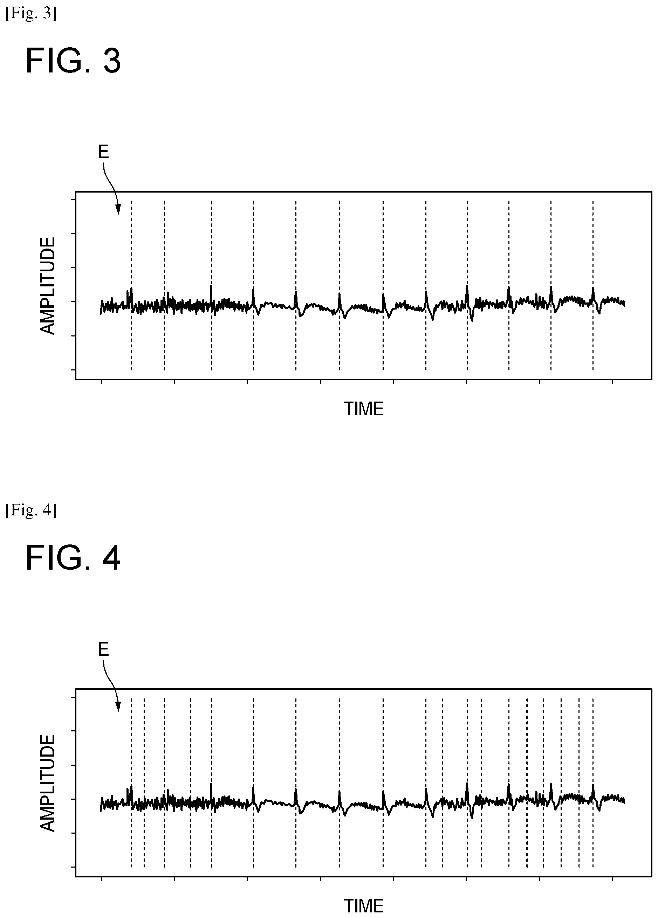

   FIG. 3 illustrates a relationship between electrocardiogram waveform data and first heart rate data.
[Fig. 4]
   FIG. 4 illustrates a relationship between the electrocardiogram waveform data and second heart rate data.
[Fig. 5]

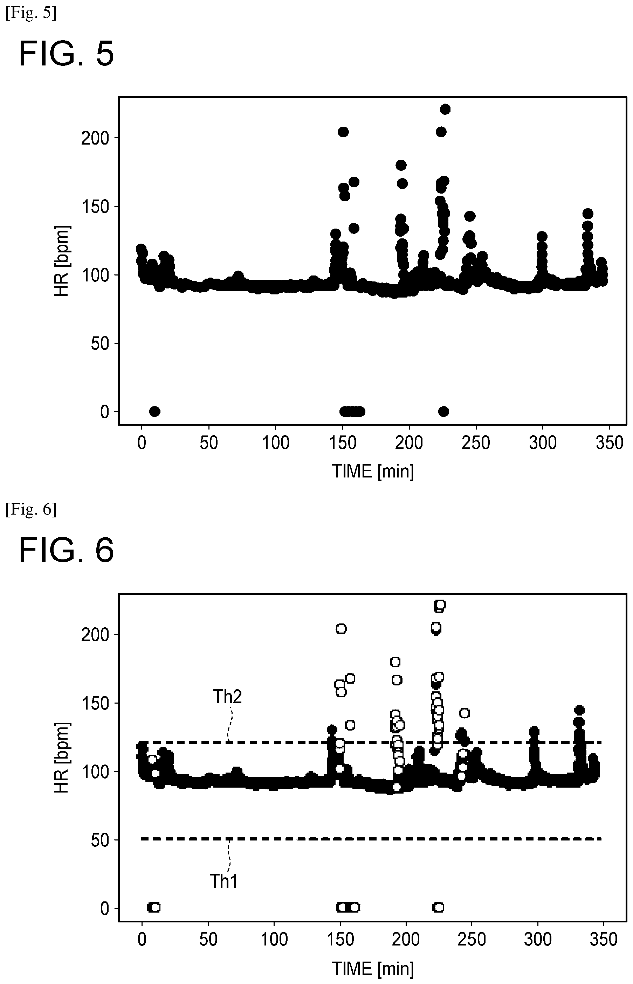

   FIG. 5 illustrates data indicating a temporal change in a heart rate acquired from a subject.
[Fig. 6]
   FIG. 6 illustrates data indicating a temporal change in the heart rate having processed by the predictor.
[Fig. 7]

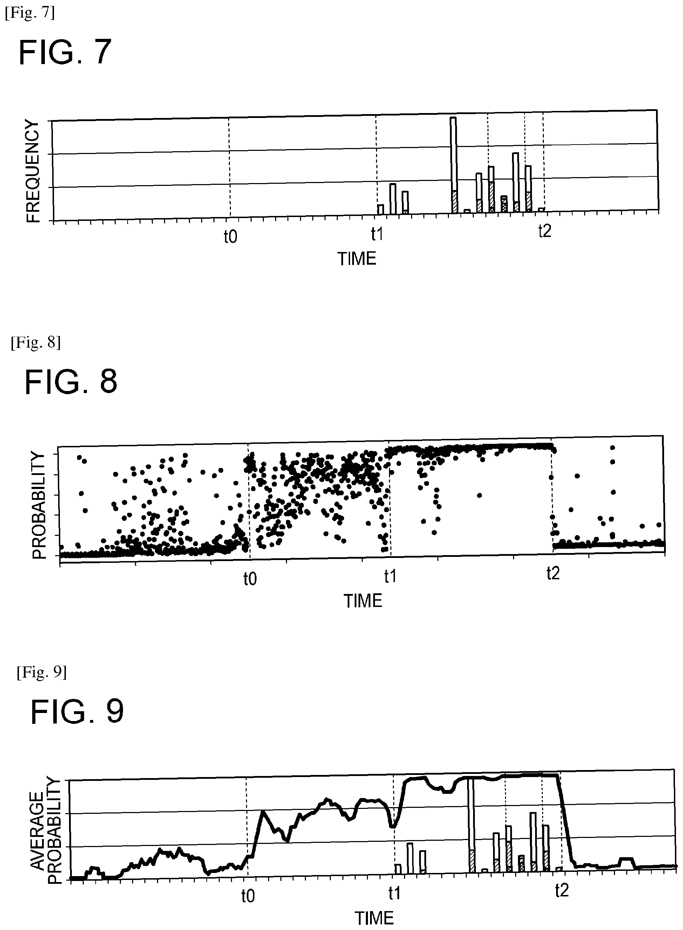

   FIG. 7 is a diagram for explaining an exemplary operation performed by the processor of FIG. 1.
[Fig. 8]
   FIG. 8 is a diagram for explaining the exemplary operation.
[Fig. 9]
   FIG. 9 is a diagram for explaining the exemplary operation.
[Fig. 10]

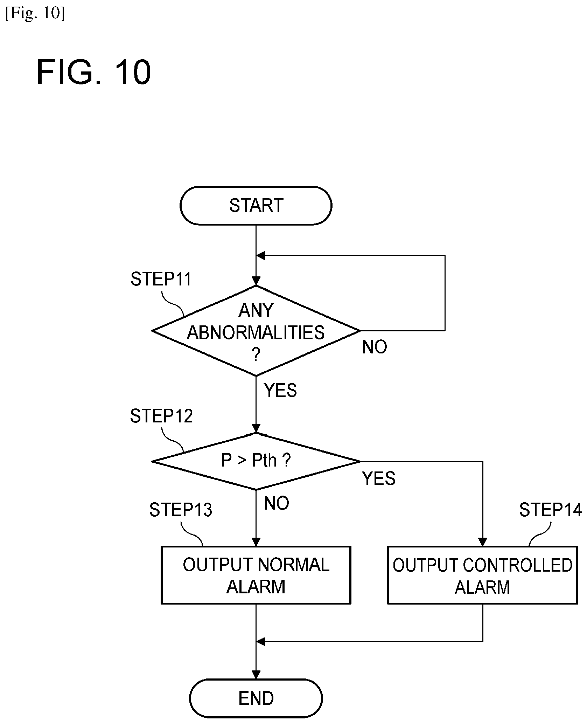

   FIG. 10 is a diagram for explaining another exemplary operation performed by the processor of FIG. 1.

### [Description of Embodiments]

Examples of embodiments will be described in detail below with reference to the accompanying drawings.

FIG. 1 illustrates a functional configuration of a physiological information acquisition device 10 according to an embodiment. The physiological information acquisition device 10 is a device configured to acquire a physiological parameter of a subject 30 through a sensor 20. Examples of the sensor 20 include an electrode for acquiring a heart rate, an electrode for acquiring an impedance respiration rate, a catheter for acquiring an invasive blood pressure value, a probe for acquiring a transcutaneous arterial oxygen saturation (SpO2) or a pulse rate, a flow sensor for acquiring a respiratory carbon dioxide concentration, and the like.

The physiological information acquisition device 10 includes a notifier 11. The notifier 11 is configured to be able to output an alarm A. The alarm A may include an alarm (a so-called technical alarm) indicating that the physiological parameter is not normally acquired, and an alarm (a so-called physiological alarm) indicating that at least one of the physiological parameter value and the measured waveform of the physiological parameter is abnormal. The alarm A includes at least one of a visual alarm, an audible alarm, and a haptic alarm. The notifier 11 is an example of an output device.

The physiological information acquisition device 10 includes a processing device 12. The processing device 12 includes a reception interface 121, a processor 122, and an output interface 123.

The reception interface 121 is configured to receive waveform data W corresponding to a measured waveform of the physiological parameter of the subject 30 from the sensor 20. The waveform data W may be in the form of analog data or digital data. In the case where the waveform data W is in the form of analog data, the reception interface 121 is configured to be equipped with an adequate conversion circuit including an A/D converter. The processor 122 is configured to process waveform data W in the form of digital data.

The processor 122 is configured to output, from the output interface 123, first control data C1 that causes the notifier 11 to output the alarm A, based on the waveform data W. For example, when the sensor 20 deviates from a prescribed position, or when any defect occurs in the operation of the sensor 20, a distortion occurs in an adequate measured waveform, or the level of the signal is degraded. In a case where the processor 122 determines that the waveform data W corresponds to such a state, the processor 122 transmits the first control data C1 from the output interface 123 to the notifier 11.

The first control data C1 transmitted to the notifier 11 may be in the form of analog data or digital data. In the case where the first control data C1 is in the form of analog data, the output interface 123 is configured to be equipped with an adequate conversion circuit including a D/A converter.

The processor 122 may be implemented by a general-purpose microprocessor that operates in cooperation with a general-purpose memory. Examples of the general-purpose microprocessor include a CPU, an MPU, and a GPU. Examples of the general-purpose memory include a ROM and a RAM. In this case, a computer program for executing processing described later may be stored in the ROM. The ROM is an example of a non-transitory computer-readable medium having stored a computer program. The processor designates at least a part of the computer program stored in the ROM, loads the designated part in the RAM, and executes the above-described processing in cooperation with the RAM. The above-described computer program may be pre-installed in a general-purpose memory, or downloaded from an external server device via a communication network, and is then installed in the general-purpose memory. In this case, the external server device is an example of the non-transitory computer-readable medium having stored therein a computer program.

The processor 122 may be realized by a dedicated integrated circuit capable of executing the above-described computer program, such as a microcontroller, an ASIC, and an FPGA. In this case, the above-described computer program is pre-installed in the memory element included in the dedicated integrated circuit. The storage element is an example of the non-transitory computer-readable medium having stored therein a computer program. The processor 122 may also be implemented by a combination of the general-purpose microprocessor and the dedicated integrated circuit.

The processing device 12 includes a predictor 124. The predictor 124 is configured to, based on the waveform data W, predict a probability P that the value of the physiological parameter is erroneously calculated. The predictor 124 is implemented as a learned model or classifier that is created by machine learning described later.

With reference to FIG. 2, a method of creating the predictor 124 that can predict a probability that the heart rate is erroneously calculated based on a measured waveform of an electrocardiogram will be described as an example.

First, a process of acquiring first heart rate data H1 from electrocardiogram waveform data E is performed in STEP 1. The first heart rate data H1 is data indicating a value of the heart rate that is correctly calculated from the electrocardiogram waveform data E. The electrocardiogram waveform data E is an example of the waveform data W. The heart rate is an example of the physiological parameter.

FIG. 3 illustrates a relationship between the electrocardiogram waveform data E and the first heart rate data H1. The electrocardiogram waveform data E represents a temporal change of the physiological electrical activity of the heart of the subject. The electrocardiogram waveform data E changes in accordance with the cardiac beat. The first heart rate data H1 is calculated by counting the number of changes per minute. The dashed lines extending in the vertical direction in FIG. 3 indicate portions that are used for counting the heart rate in the electrocardiogram waveform data E. In FIG. 2B, 12 cardiac beats are counted during a time period when the waveform is displayed, for example, 10 seconds.

The count of the heart rate corresponding to the first heart rate data H1 may be performed by visually observing the electrocardiogram waveform data E, or may be performed by using an adequate counting software. As the electrocardiogram waveform data E, waveform data that is actually acquired from the subject by using an electrocardiogram sensor or the like may be used, or waveform data that is disclosed as a database for evaluating the performance of an electrocardiograph may be used.

Subsequently or in parallel, a process of acquiring the second heart rate data H2 from the electrocardiogram waveform data E is performed (STEP 2 in FIG. 2). The second heart rate data H2 is data indicating a value of the heart rate that is automatically calculated from the electrocardiogram waveform data E by a specific heart rate calculation algorithm. The heart rate calculation algorithm may be executed by, for example, the processor 122 of the processing device 12 that is incorporated in the physiological information acquisition device 10. The heart rate calculation algorithm is configured to automatically calculate a heart rate based on, for example, the feature of the waveform profile in the inputted electrocardiogram waveform data E.

FIG. 4 illustrates a relationship between the electrocardiogram waveform data E and the second heart rate data H2. The electrocardiogram waveform data E illustrated in FIG. 4 is the same as the electrocardiogram waveform data E illustrated in FIG. 3. In the example illustrated in FIG. 4, 19 cardiac beats are counted instead of 12, based on the same electrocardiogram waveform data E. This fact means that the heart rate calculation algorithm that is used in acquiring the second heart rate data H2 may perform the count based on the cardiac beat that is erroneously detected.

FIG. 5 illustrates a temporal change of the heart rate that is automatically calculated by inputting the electrocardiogram waveform data E that is actually acquired from the subject, to the above-described heart rate calculation algorithm. Each of the points included in the graph corresponds to a heart rate detected within a prescribed time period, for example, one minute.

In FIG. 6, values that are erroneously calculated from the heart rate values illustrated in FIG. 5 are indicated by white circles. One technique that may exclude erroneously calculated heart rate data is to set a threshold range for the heart rate. For example, the heart rate data in which the heart rate does not fall between a lower limit threshold value Th1 and an upper limit threshold value Th2 may be excluded. However, as illustrated in FIG. 6, even within the threshold range, there may be erroneously calculated heart rate data. Conversely, even outside the threshold range, there may be correctly calculated heart rate data. The former is based on an erroneous analysis of the heart rate calculation algorithm, and the latter corresponds to a case where the heart rate is increased due to some physiological phenomenon in the subject. The erroneous analysis of the heart rate calculation algorithm may be caused by, for example, a phenomenon (so-called double count) in which a T wave with a large amplitude is erroneously recognized as a QRS wave, thereby counting the heart rate excessively. The erroneous analysis of the heart rate calculation algorithm may be caused by external noise such as body motions of the subject, or the degradation or dropout of the electrode attached to the subject.

Subsequently, the second heart rate data H2 corresponding to the heart rate that is automatically calculated by the heart rate calculation algorithm is collated with the first heart rate data H1, whereby a process of creating training data T including a training label indicating whether the heart rate is correct or incorrect is performed (STEP 3 in FIG. 2).

In a case where the first heart rate data H1 illustrated in FIG. 3 and the second heart rate data H2 illustrated in FIG. 4 are subjected to the collation, the training data T includes a training label indicating that the heart rate corresponding to the second heart rate data H2 is incorrect.

In order to include the training label indicating that the answer is correct, it is not necessarily required that the heart rate corresponding to the first heart rate data H1 and the heart rate corresponding to the second heart rate data H2 completely coincide with each other. As long as the error of the heart rate corresponding to the second heart rate data H2 with respect to the heart rate corresponding to the first heart rate data H1 is within an allowable range, the training data T may include a training label indicating a correct answer.

Namely, the heart rate that is automatically calculated by the heart rate calculation algorithm is used for collation with a heart rate that is known to be correctly calculated. Accordingly, the training label that is obtained as a result of the collation may reflect a tendency or habit that the heart rate calculation algorithm correctly or erroneously calculates the heart rate with respect to the inputted electrocardiogram waveform data E.

Subsequently, the machine learning of the predictor 124 is performed based on the combination of the electrocardiogram waveform data E and the training data T (STEP 4 in FIG. 2). In the machine learning, a well-known technique related to the supervised learning is appropriately used. Namely, the predictor 124 learns a feature quantity of the electrocardiogram waveform data E in which the heart rate is erroneously calculated, and modifies the calculation parameter so as to output prediction data indicating that a probability P that the heart rate is erroneously calculated is high when the electrocardiogram waveform data E having such a feature quantity is inputted. The modification of the calculation parameter may be performed by an operator of the machine learning, or may be performed by the predictor 124 itself through the machine learning. The prediction data may indicate the calculated probability P itself, or may be associated with a score corresponding to the probability P (for example, any of the values 1 to 5), or the like.

As illustrated in FIG. 1, the processor 122 is configured to output second control data C2 from the output interface 123 based on the probability P that is calculated by the predictor 124. The second control data C2 is configured to cause the notifier 11 to output a preliminary notification N described later. The second control data C2 transmitted to the notifier 11 may be in the form of analog data or digital data. In the case where the second control data C2 is in the form of analog data, the output interface 123 is configured to be equipped with an adequate conversion circuit including a D/A converter.

A preliminary notification N that is performed by the physiological information acquisition device 10 will be described in detail with reference to FIGS. 7 to 9.

FIG. 7 is a bar graph illustrating the frequency of alarms outputted while an electrocardiogram of a subject is acquired with a conventional physiological information acquisition device. The alarm is outputted to indicate that the heart rate is not normally acquired. The alarm is outputted due to body movements of the subject, degradation positional deviation, dropout, or the like of an electrode attached to the subject. The blank bar represents an alarm with a duration of less than 5 seconds. The hatched bar indicates an alarm with a duration of no less than 5 seconds and less than 30 seconds. The cross-hatched bar indicates an alarm with a duration of no less than 30 seconds.

In the example illustrated in FIG. 7, an alarm output begins from a time point t1. It is understood that the above-described cause that hinders the normal acquisition of the heart rate occurs. It is understood that, with the elapse of time, the frequency of alarm outputs increases, as well as the number of alarms with a long duration increases, whereby the situation is getting worse. At a time point t2, the electrode is replaced or rearranged, whereby the alarm output is stopped.

FIG. 8 is a graph plotting the probabilities P that are outputted from the predictor 124 in a case where the electrocardiogram waveform that is acquired in the example illustrated in FIG. 7 is used as an input to the predictor 124 according to the present embodiment. FIG. 9 is a graph illustrating a temporal change of a smoothed probability P that is obtained by calculating a temporal moving average value of the plots illustrated in FIG. 8, while being superimposed on the bar graph of FIG. 7. It is evident that there is a correlation between the time period in which the alarm is outputted and the time period in which the probability P that the heart rate is erroneously calculated (the output from the predictor 124) is high.

The inventors of the present application paid attention to a time period that precedes the time point t1. The probability P is not suddenly transited from a state where it changes with lower values before a time point t0 preceding the time point t1 to a state where it changes with higher values after the time point t1. Rather, the frequency that the probability P takes a relatively high value gradually increases during a time period from a time point t0 to the time point t1. In other words, there is a time period in which a transition is occurred from a state where the heart rate is normally acquired to a state where the heart rate is not normally acquired without the alarm output.

In view of the above, the processor 122 is configured to cause the notifier 11 to output a preliminary notification N based on the rising trend of the probability P that is calculated by the predictor 124. For example, in a case where the frequency at which the probability P exceeds a prescribed probability threshold (the number of times per unit time) exceeds a prescribed frequency threshold, the processor 122 may cause the notifier 11 to output the preliminary notification N. Alternatively, the processor 122 may cause the notifier 11 to output the preliminary notification N in a case where the cumulative number of times when the probability P exceeds the prescribed probability threshold exceeds a prescribed number of times threshold. Alternatively, the processor 122 may cause the notifier 11 to output the preliminary notification N in a case where the temporal moving average value of the probability P illustrated in FIG. 9 exceeds a prescribed probability threshold.

The preliminary notification N notifies at least one of the quality of the waveform data W, the state of the sensor 20, and an action shall be taken by a user. The preliminary notification N includes at least one of a visual notification, an audible notification, and a haptic notification.

In the case of the example illustrated in FIGS. 8 and 9, the increase in the probability P that the heart rate is erroneously calculated may be caused by a deviation of the electrocardiogram waveform from the normal state due to the body motions, the degradation or the positional deviation of the electrode, or the like. For example, in the preliminary notification N, a message such as "The waveform is disturbed," "The body motions are increasing," or "Check the state of the electrode" may be provided to the user through a display or a voice.

According to the above configuration, it is possible to predict a sign reaching a state where alarms are frequently outputted, thereby prompting the user to take any suitable actions. Accordingly, it is possible to suppress the occurrence of a situation where the user is forced to take an unnecessary action by an alarm.

It is preferable that the condition for outputting the preliminary notification N is determined such that the preliminary notification N is performed before reaching the state where the alarm A is outputted by the notifier 11. In the example illustrated in FIGS. 8 and 9, a condition as for the probability P is determined such that the preliminary notification N is performed before the time point t1. By prompting the user to take a suitable action by the preliminary notification N that precedes the output of the alarm A, it is possible to increase a possibility that the output of the alarm A that imposes a certain degree of tension on the user can be avoided.

The above-described electrocardiogram waveform data E corresponds to a waveform that is obtained by synthesizing a plurality of lead waveforms, or a waveform that is obtained by simply acquiring a single lead (generally, the lead II). However, the predictor 124 may be created so as to predict a probability that the heart rate is erroneously calculated based on each of the lead waveforms. According to the above configuration, it is possible to carefully manage the state of each electrode from which a lead waveform is acquired, and it is possible to easily suppress the occurrence of a situation where the user is forced to take an unnecessary action by an alarm.

Next, another exemplary configuration of the physiological information acquisition device 10 will be described with reference to FIG. 10. In this example, the processor 122 of the processing device 12 determines whether there is an abnormality in at least one of the value and the measured waveform of the physiological parameter based on the waveform data W received by the reception interface 121 (STEP 11). The process is repeated until it is determined that at least one of the value and the measured waveform of the physiological parameter is abnormal (NO in STEP 11).

When it is determined that at least one of the value and the measured waveform of the physiological parameter is abnormal (YES in STEP 11), the processor 122 determines whether or not the probability P that the value of the physiological parameter is erroneously calculated that is predicted by the predictor 124 exceeds the probability threshold Pth (STEP 12).

When it is determined that the probability P does not exceed the probability threshold Pth (NO in STEP 12), the processor 122 outputs the first control data C1 that causes the notifier 11 to output a normal alarm A that is prescribed in advance, from the output interface 123 (STEP 13). As described above, the alarm A may be outputted in at least one of a visual manner, an audible manner, and a haptic manner.

When it is determined that the probability P exceeds the probability threshold Pth (YES in STEP 12), the processor 122 outputs a third control signal CS3 that causes the notifier 11 to output a controlled alarm A' from the output interface 123 (STEP 14). As used herein, the expression "controlled alarm" or "control the output of an alarm" means applying a somewhat change to a normal alarm A assigned to a certain situation. Examples of the change include a change of the urgency of the alarm, a stop of the alarm output, an addition of the information to be provided, and an omission of the information to be provided. Namely, the expression "output of a controlled alarm" means to include a situation that the normal alarm is not outputted.

For example, the alarm A may include levels of "emergency," "alert," and "caution," depending on the degree of urgency. In the case where the electrocardiogram waveform is to be acquired, an alarm at the "emergency" level may be assigned to a situation where ventricular fibrillation or ventricular tachycardia occurs. An alarm at the "alert" level may be assigned to a situation where the heart rate is abnormal or a ventricular premature beat occurs. An alarm at the "caution" level may be assigned to other anomalies.

The ways of the notification are different between the levels. For example, in a case where the alarm A includes a visual alarm, colors and symbols that are displayed are different from each other. In a case where the alarm A includes an audible alarm, volumes, intervals, durations or the like of the sounds to be outputted are different from each other.

For example, in a case where it is determined that ventricular fibrillation occurs based on the electrocardiogram waveform data E, and the probability P that the heart rate is erroneously calculated does not exceed the probability threshold Pth (NO in STEP 12), the processor 122 causes the notifier 11 to output an alarm A of the "emergency" level that is assigned to the ventricular fibrillation (STEP 13).

On the other hand, in a case where it is determined that ventricular fibrillation occurs based on the electrocardiogram waveform data E, while the probability P that the heart rate is erroneously calculated exceeds the probability threshold Pth (YES in STEP 12), the processor 122 applies the change to the alarm A at the "emergency" level assigned to the ventricular fibrillation, and outputs a controlled alarm A' (STEP 14).

Even when any abnormality is observed in the electrocardiogram waveform that is received by the reception interface 121, in a case where the probability P that the heart rate that is erroneously calculated that is predicted by the predictor 124 based on the same electrocardiogram waveform is high, it is highly probable that probability that the abnormality is erroneously detected. In such a case, by applying some change to the alarm A that is to be outputted by the notifier 11, it is possible to cause a user to recognize that there is a possibility that an abnormality is erroneously detected. Accordingly, it is possible to suppress the occurrence of a situation where the user is forced to take an unnecessary action by an alarm.

The processor 122 may cause the notifier 11 to output an index indicating the probability P that is predicted by the predictor 124. The index may be provided using at least one of a visual index, an audible index, and a haptic index.

According to the above configuration, the user can more reliably recognize that there is a possibility that an abnormality is erroneously detected.

In a case where the alarm A includes a visual alarm and an audible alarm, the processor 122 may output a controlled alarm A' in which the urgency of at least one of the visual alarm and the audible alarm is lowered.

For example, in a case where the normal alarm A includes a visual alarm and an audible alarm that are assigned to the "emergency" level, at least one of the visual alarm and the audible alarm may be changed to one that is assigned to the "alert" level or the "caution" level. For example, the sound assigned to the "emergency" level is changed to the sound assigned to the "alert" level while the color display assigned to the "emergency" level is maintained.

According to the above configuration, the user's attention can be called by not canceling the alarm output while the tension involved in the alarm output with a higher urgency can be mitigated. In a case where the urgency of one of the audible alarm and the audible alarm is lowered, it is also possible to cause the user to recognize a possibility that the abnormality is erroneously detected based on a fact that the combination is different from that of the normal alarms.

The processor 122 may cause the notifier 11 to provide additional information corresponding to the fact that the probability P that the physiological parameter is erroneously detected that is predicted by the predictor 124 exceeds the probability threshold Pth, while maintaining the urgency of the normal alarm A. The provision of the additional information may be performed by using at least one of a visual manner, an audible manner, and a haptic manner.

According to the above configuration, the user's attention can be called by not canceling the alarm output while the tension involved in the alarm output with a higher urgency can be mitigated by causing the user to recognize the possibility that the abnormality is erroneously detected through the provision of the additional information.

The above embodiments are illustrative only to facilitate an understanding of the presently disclosed subject matter. The configuration according to the above embodiment can be appropriately modified or improved without departing from the gist of the presently disclosed subject matter.

In the above embodiment, in order to create the predictor 124 for predicting the probability that the heart rate is erroneously calculated, the first heart rate data H1 and the second heart rate data H2 are acquired by using the common electrocardiogram waveform data E. As described above, the first heart rate data H1 is data indicating the heart rate that is correctly calculated from the electrocardiogram waveform data E. The second heart rate data H2 is data indicating the heart rate that is calculated by a specific heart rate calculation algorithm, and may include a heart rate that is erroneously calculated.

As another example, in order to create the predictor 124 for predicting the probability that the respiration rate is erroneously calculated, the first respiration rate data and the second respiration rate data may be acquired by using common capnogram data. The capnogram data is an example of the waveform data. The respiratory rate is an example of the physiological parameter. The first respiratory rate data is data indicating the respiratory rate that is correctly calculated from the capnogram data. The second respiratory rate data is data indicating the respiratory rate that is calculated by a specific respiratory rate calculation algorithm, and may include a respiratory rate that is erroneously calculated.

The physiological parameter for which the predictor 124 predicts the probability of the erroneous calculation may be different from a physiological parameter for which an alarm is outputted. For example, the predictor 124 may be used for predicting a probability that the heart rate is erroneously calculated with respect to a configuration in which an alarm is outputted for an abnormality of an ST value, a QT interval, or a QTc in the electrocardiogram waveform data. Alternatively, the predictor 124 may be used for predicting a probability that the respiratory rate is erroneously calculated with respect to a configuration in which an alarm is outputted for an abnormality of a partial pressure of end-expiratory carbon dioxide (EtCO2) in the capnogram data.

Different types of physiological parameters may be used in the creation of the predictor 124. As an example, in order to create the predictor 124 for predicting the probability that the pulse rate is erroneously calculated, heart rate data acquired from the electrocardiogram waveform data, and pulse rate data acquired from invasive arterial pressure waveform data may be used. The electrocardiogram waveform data and the invasive arterial pressure waveform data are required to be acquired from the same subject. The heart rate data is data indicating a heart rate that is correctly calculated from the electrocardiogram waveform data. The pulse rate data is data indicating the pulse rate that is calculated by a specific pulse rate calculation algorithm, and may include a pulse rate that is erroneously calculated.

The predictor 124 that is created as described above is used for predicting a probability that an invasive arterial pressure is erroneously calculated. The invasive arterial pressure waveform data is an example of the waveform data. The invasive arterial pressure is an example of the physiological parameter.

The pulse wave waveform data may be used to acquire the pulse rate data. The pulse wave waveform data may be acquired by non-invasively measuring a temporal change of the absorbance of blood by using a pulse photometry probe or the like. Alternatively, the pulse wave waveform data may be acquired by measuring a temporal change of an inner pressure of a cuff by using a non-invasive arterial manometer or the like. In this case, the created predictor 124 is used for predicting a probability that the non-invasive arterial pressure, the pulse rate, and the transcutaneous arterial oxygen saturation (SpO2) are erroneously calculated. The pulse wave waveform data is an example of the waveform data. Each of the invasive arterial pressure, the pulse rate, and the SpO2 is an example of the physiological parameter.

As another example, in order to create the predictor 124 for predicting the probability that the respiratory rate is erroneously calculated, first respiratory rate data acquired from the impedance respiratory waveform data, and second respiratory rate data acquired from the capnogram data may be used. The impedance respiratory waveform data and the capnogram data are required to be acquired from the same subject. The first respiratory rate data is data indicating the respiratory rate that is correctly calculated from the impedance respiratory waveform data. The second respiratory rate data is data indicating the respiratory rate that is calculated by a specific respiratory rate calculation algorithm, and may include a respiratory rate that is erroneously calculated. The impedance respiration waveform data is an example of the waveform data. The respiratory rate is an example of the physiological parameter.

In the above embodiment, the notifier 11 that outputs the alarm A, the controlled alarm A', and the preliminary notification N is installed in the physiological information acquisition device 10. However, the function of the notifier 11 may be implemented by an independent output device that is capable of performing communication with the physiological information acquisition device 10 via a communication network. In this case, the processor 122 of the processing device 12 transmits the first control data C1 and the second control data C2 for causing the output device to output the alarm A, the controlled alarm A', and the preliminary notification N from the output interface 123.

In the above embodiment, both the notifier 11 and the processing device 12 are installed in the physiological information acquisition device 10. However, the processing device 12 may be installed in an external server device that can communicate with the physiological information acquisition device 10 via a communication network. In this case, the processing device 12 executes the above-described processing based on the waveform data W received from the sensor 20 or the physiological information acquisition device 10 via the communication network, transmits suitable control data via the communication network, and causes the notifier 11 to output the alarm A, the controlled alarm A', and the preliminary notification N. The device performing an operation based on the control data transmitted from the external server device may be an output device that is independent from the physiological information acquisition device 10.

The present application is based on Japanese Patent Application No. 2020-090725 filed on May 25, 2020, the entire contents of which are hereby incorporated by reference.
The following are further embodiments of the present invention:
1. A physiological information acquisition device, comprising:
   a reception interface configured to receive waveform data corresponding to a measured waveform of a physiological parameter of a subject from a sensor;
   a notifier configured to output an alarm indicating that the physiological parameter is not normally acquired;
   a processor configured to cause the notifier to output the alarm based on the waveform data; and
   a predictor configured to predict, based on the waveform data, a probability that the physiological parameter is erroneously calculated,
   wherein the processor is configured to cause the notifier to perform a notification of at least one of a quality of the waveform data, a state of the sensor, and an action shall be taken by a user, based on the probability.
2. The physiological information acquisition device according to embodiment 1, wherein the processor is configured to cause the notifier to perform the notification based on a frequency that the probability exceeds a threshold value.
3. The physiological information acquisition device according to embodiment 1 or 2, wherein the processor is configured to be able to cause the notifier to perform the notification before the alarm is outputted.
4. The physiological information acquisition device according to any one of embodiments 1 to 3,
   wherein the physiological parameter is a heart rate.
5. The physiological information acquisition device according to embodiment 4,
   wherein the sensor includes a plurality of electrodes adapted to be attached to the subject to measure an electrocardiogram; and
   wherein the predictor is configured to calculate the probability for the waveform data associated with each of lead waveforms included in the electrocardiogram.
6. A processing device configured to process physiological information of a subject, comprising:
   a reception interface configured to receive waveform data corresponding to a measured waveform of a physiological parameter of the subject from a sensor;
   a processor configured to cause, based on the waveform data, an output device to output an alarm indicating that the physiological parameter is not normally acquired; and
   a predictor configured to predict, based on the waveform data, a probability that the physiological parameter is erroneously calculated,
   wherein the processor is configured to cause the output device to perform a notification of at least one of a quality of the waveform data, a state of the sensor,
   and an action shall be taken by a user, based on the probability.
7. A non-transitory computer-readable medium having stored a computer program adapted to be executed by a processor of a processing device configured to process physiological information of a subject, the computer program being configured to, when executed, the processing device to:
   receive waveform data corresponding to a measured waveform of a physiological parameter of the subject from a sensor;
   cause, based on the waveform data, an output device to output an alarm indicating that the physiological parameter is not normally acquired;
   predict, based on the waveform data, a probability that the physiological parameter is erroneously calculated; and
   cause the output device to perform a notification of at least one of a quality of the waveform data, a state of the sensor, and an action shall be taken by a user, based on the probability.
8. A physiological information acquisition device, comprising:
   a reception interface configured to receive waveform data corresponding to a measured waveform of a physiological parameter of a subject from a sensor;
   a notifier configured to output an alarm indicating an abnormality of at least one of a value and the measured waveform of the physiological parameter;
   a processor configured to cause the notifier to output the alarm based on the waveform data; and
   a predictor configured to predict, based on the waveform data, a probability that the physiological parameter is erroneously calculated,
   wherein the processor is configured to control an output of the alarm in a case where the probability exceeds a threshold value.
9. The physiological information acquisition device according to embodiment 8, wherein the notifier is configured to notify an index corresponding to the probability.
10. The physiological information acquisition device according to embodiment 8 or 9,
   wherein the alarm includes a visual alarm and an audible alarm; and
   wherein the processor is configured to control the alarm so as to lower an urgency of at least one of the visual alarm and the audible alarm.
11. The physiological information acquisition device according to embodiment 8 or 9, wherein the processor is configured to control the alarm so as to provide an additional notification corresponding to a fact that the probability exceeds the threshold value while maintaining an urgency of the alarm.
12. A processing device configured to process physiological information of a subject, comprising:
   a reception interface configured to receive waveform data corresponding to a measured waveform of a physiological parameter of a subject from a sensor;
   a processor configured to cause, based on the waveform data, an output device to output an alarm indicating an abnormality of at least one of a value and the measured waveform of the physiological parameter; and
   a predictor configured to predict, based on the waveform data, a probability that the physiological parameter is erroneously calculated,
   wherein the processor is configured to control an output of the alarm in a case where the probability exceeds a threshold value.
13. A non-transitory computer-readable medium having stored a computer program adapted to be executed by a processor of a processing device configured to process physiological information of a subject, the computer program being configured to, when executed, the processing device to:
   receive waveform data corresponding to a measured waveform of a physiological parameter of a subject from a sensor;
   cause, based on the waveform data, an output device to output an alarm indicating an abnormality of at least one of a value and the measured waveform of the physiological parameter;
   predict, based on the waveform data, a probability that the physiological parameter is erroneously calculated; and
   control an output of the alarm in a case where the probability exceeds a threshold value.

## Claims

1. A physiological information acquisition device, comprising:
a reception interface configured to receive waveform data corresponding to a measured waveform of a physiological parameter of a subject from a sensor;
a notifier configured to output an alarm indicating an abnormality of at least one of a value and the measured waveform of the physiological parameter;
a processor configured to cause the notifier to output the alarm based on the waveform data; and
a predictor configured to predict, based on the waveform data, a probability that the physiological parameter is erroneously calculated,
wherein the processor is configured to control an output of the alarm in a case where the probability exceeds a threshold value.

2. The physiological information acquisition device according to claim 1,
wherein the notifier is configured to notify an index corresponding to the probability.

3. The physiological information acquisition device according to claim 1 or 2,
wherein the alarm includes a visual alarm and an audible alarm; and
wherein the processor is configured to control the alarm so as to lower an urgency of at least one of the visual alarm and the audible alarm.

4. The physiological information acquisition device according to claim 1 or 2,
wherein the processor is configured to control the alarm so as to provide an additional notification corresponding to a fact that the probability exceeds the threshold value while maintaining an urgency of the alarm.

5. A processing device configured to process physiological information of a subject, comprising:
a reception interface configured to receive waveform data corresponding to a measured waveform of a physiological parameter of a subject from a sensor;
a processor configured to cause, based on the waveform data, an output device to output an alarm indicating an abnormality of at least one of a value and the measured waveform of the physiological parameter; and
a predictor configured to predict, based on the waveform data, a probability that the physiological parameter is erroneously calculated,
wherein the processor is configured to control an output of the alarm in a case where the probability exceeds a threshold value.

6. A non-transitory computer-readable medium having stored a computer program adapted to be executed by a processor of a processing device configured to process physiological information of a subject, the computer program being configured to, when executed, the processing device to:
receive waveform data corresponding to a measured waveform of a physiological parameter of a subject from a sensor;
cause, based on the waveform data, an output device to output an alarm indicating an abnormality of at least one of a value and the measured waveform of the physiological parameter;
predict, based on the waveform data, a probability that the physiological parameter is erroneously calculated; and
control an output of the alarm in a case where the probability exceeds a threshold value.
